(19) Europäisches Patentamt
European Patent Office
Office européen des brevets

(11) Publication number: **0 265 686 B1**

(12) **EUROPEAN PATENT SPECIFICATION**

(45) Date of publication of patent specification: **27.11.91**

(51) Int. Cl.[5]: **C07C 323/09**, C07D 295/22, G01N 33/52, C12Q 1/32

(21) Application number: **87114167.7**

(22) Date of filing: **29.09.87**

(54) **Test agents and indicators for the detection of thiol groups and processes for their preparation.**

(30) Priority: **10.10.86 DE 3634525**

(43) Date of publication of application:
**04.05.88 Bulletin 88/18**

(45) Publication of the grant of the patent:
**27.11.91 Bulletin 91/48**

(84) Designated Contracting States:
**DE FR GB**

(56) References cited:

**CHEMICAL ABSTRACTS, vol. 90, no. 19, 7th May 1979, page 575, no. 151800v, Columbus, Ohio, US; & RO-A-63 418 (INTERPRINDEREA DE MEDICAMENTE "TERAPIA") 30-01-1978**

**ARCHIVES OF BIOCHEMISTRY AND BIOPHYSICS, vol. 82, 1959, pages 70-77; G.L. ELLMAN: "Tissue sulfhydryl groups"**

(73) Proprietor: **MILES INC.**
**1127 Myrtle Street**
**Elkhart Indiana 46514(US)**

(72) Inventor: **Heidenreich, Holger, Dr.**
**Andreas-Gryphius-Strasse 22**
**W-5000 Köln 80(DE)**
Inventor: **Wehling, Klaus, Dr.**
**Pahlke Strasse 5**
**W-5600 Wuppertal 1(DE)**

(74) Representative: **Dänner, Klaus, Dr. et al**
**c/o Bayer AG Konzernverwaltung RP Patentabteilung**
**W-5090 Leverkusen 1 Bayerwerk(DE)**

**EP 0 265 686 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to indicators and agents for the detection of thiol groups which are present in a system or are formed in a step prior to the detection reaction. Processes for the preparation of the indicators are also described. The thiol groups can be formed by chemical reactions, such as, for example, reduction of disulphides, or biochemical reactions. The preferred biochemical reactions which lead to the formation of thiol groups are illustrated by the following equation:

a)

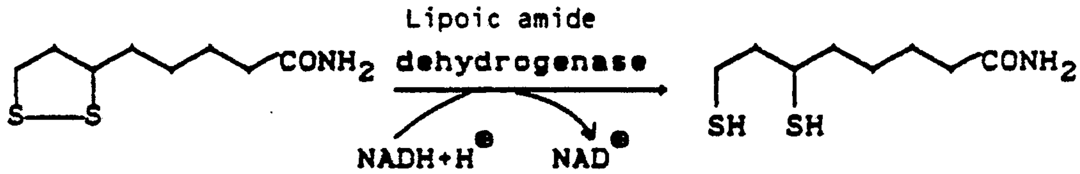


b)

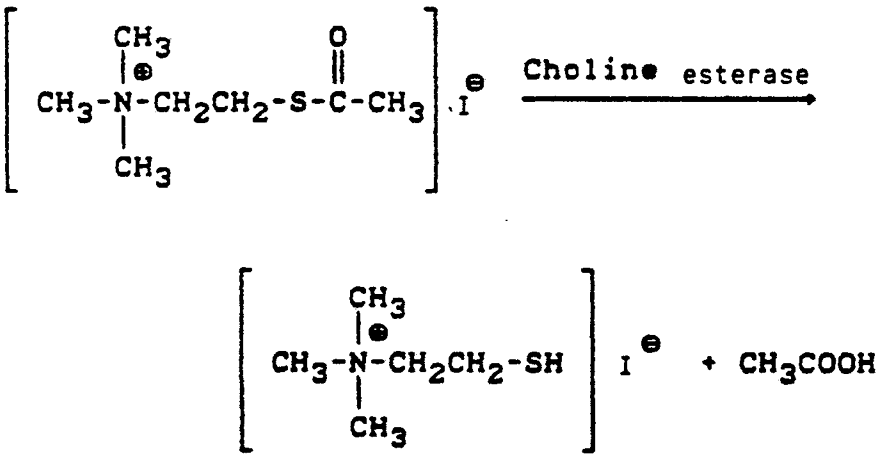


Numerous methods are known for the detection of compounds containing thiol groups. A method which is mentioned particularly frequently is that described by G.H. Ellman in "Arch. Biochem. Biophys. 1959, 82, 70-77", which is based on the formation of the yellow anion of 3-mercapto-6-nitro-benzoic acid formed by reaction between a thiol and 3,3'-dithio-bis-6-nitro-benzoic acid (Ellman's reagent).

The reaction is sensitive and fast. However, the anion of Ellman's reagent is very sensitive towards oxidation and the coloration formed is therefore unstable. Furthermore, Ellmann's reagent is very sparingly soluble in polar solvents, such as, for example, water, and therefore can be used to only a limited degree for some thiol solutions, especially those which are prepared biochemically in aqueous systems.

The compounds according to the invention, of the general formula I

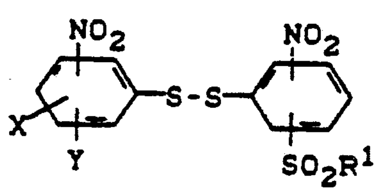

I

wherein

X represents hydrogen or $-SO_2R^1$,

Y represents hydrogen or $-NO_2$,

$R^1$ represents $-O-R^2$ or

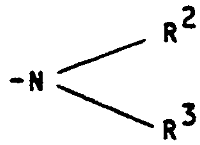

and
$R^2$ and $R^3$ independently of one another represent hydrogen or $C_1$-$C_4$-alkyl, which can be substituted by halogen, -COOH, cyano, $C_1$-$C_4$-alkoxy, hydroxyl, $-OSO_3H$, $-SO_3H$ or amino, it being possible for the amino group in turn to be substituted by $C_1$-$C_4$-alkyl which is optionally substituted by halogen, hydroxyl, cyano, $-OSO_3H$ or $-SO_3H$, or by phenyl or benzyl groups, or $R^2$ and $R^3$, together with the N atom, represent a pyrrolidine, pyrazoline, piperidine, piperazine or morpholine ring, which can be substituted by $C_1$- to $C_4$-alkyl or phenyl,
do not have these disadvantages.

Particularly preferred compounds are those of the general formula II

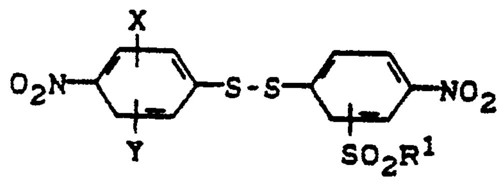

II

wherein
X, Y and $R^1$ have the abovementioned meaning.

Compounds which are of particular interest are those of the general formula III

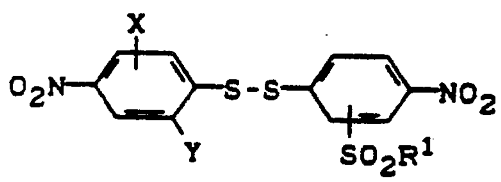

III

wherein
X, Y and $R^1$ have the abovementioned meaning.

Especially preferred compounds are those of the general formula IV

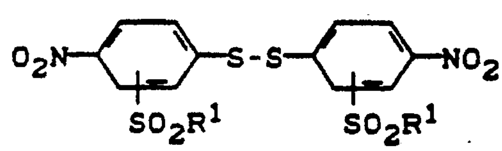

IV

wherein
$R^1$ has the abovementioned meaning.

If $R^1$ represents $-O-R^2$ or

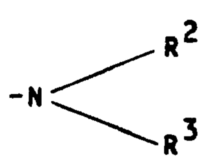

wherein
$R^2$ is other than hydrogen but $R^2$ and $R^3$ otherwise have the same meaning as mentioned above, the compounds as such are new.

Compounds which are of special interest are those of the general formula V

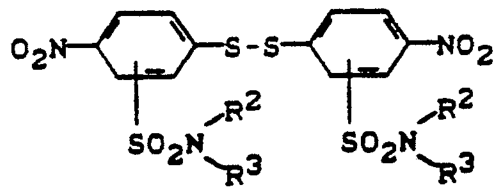

V

wherein
$R^2$ and $R^3$ have the meaning given in the case of formula I.

Especially preferred compounds are those of the general formula VI

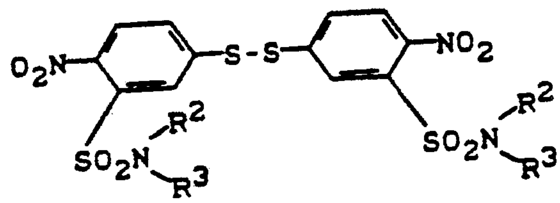

VI

wherein
$R^2$ and $R^3$ have the meaning described in the case of formula I.

The compounds I are prepared by processes which are known in the individual steps

The compounds of the formula I are prepared by a process in which a chloro-nitro-benzenesulphonyl chloride of the formula

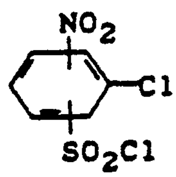

is converted with an amine or alcoholate of the formula H-$R^1$,
wherein
$R^1$ has the abovementioned meaning,
into the corresponding chloro-nitro-benzenesulphonic acid derivative and a chloride/sulphide replacement reaction is then carried out in the presence of $Na_2S$, followed by oxidation, a symmetric disulphide being obtained. Asymmetric disulphides of the formula I are prepared by a process in which a mercapto-nitro-benzene-sulphonic acid derivative obtained by chloride/sulphide replacement reaction, of the formula

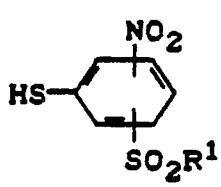

wherein
$R^1$ has the abovementioned meaning,
is reacted with a substituted phenylsulphenyl chloride of the formula

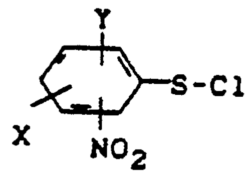

wherein

X and Y have the abovementioned meaning.

The following equation shows both routes of the synthesis:

a) Symmetric disulphides

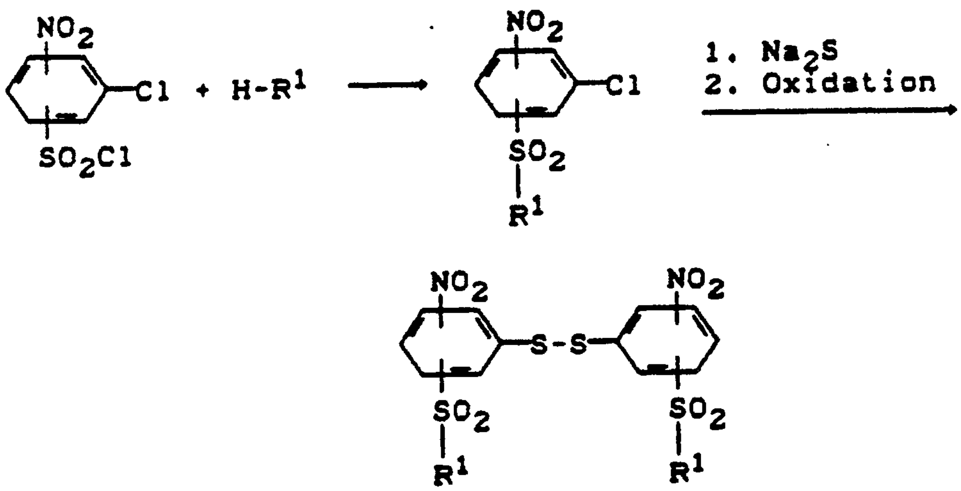


b) Asymmetric disulphides

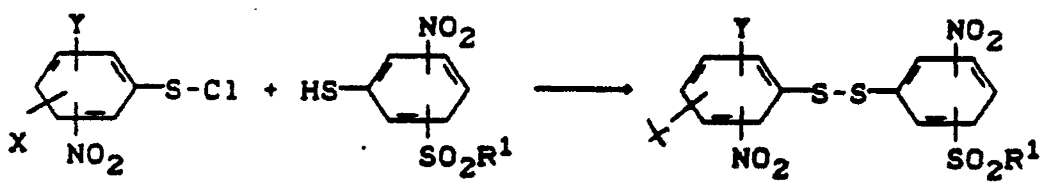

The compounds of the general formula I are particularly suitable for use as indicators in test agents for thiols or thiol precursors, such as, for example, lipoic amide, glutathione or coenzyme A.

Test agent or test system in the context of the present invention are to be understood, for example, as those which can be measured in a cell. In addition to the compounds of the general formula (I), the test agents contain all the reagents necessary for the of the particular analysis substance, such as enzymes, substrates, coenzymes, effectors, antigens, antibodies and the like. These test agents can furthermore also contain non-reacting substances, such as, for example, buffers, wetting agents and stabilizers. Enzymes which form thiol groups and which may be mentioned are thioglycosidases, thioether hydrolases and also esterases, such as, for example, choline esterase. There may also be mentioned enzymes which form thiol groups with the aid of NADH and NADPH as the coenzyme, such as lipoic amide dehydrogenase and glutathione reductase. The enzymes last mentioned can be used in test systems in which NADH or NADPH is formed. Such reactions are the known dehydrogenase-catalyzed oxidations of substrates. From the reagents and substances mentioned, it is possible to prepare reagent combinations which are mixed as a solution or powder or are in the form of tablets or a lyophilisate. The reagent combination (if it is not already present as a solution) is taken up in water or another suitable solvent and a reagent solution is prepared. If the reagent combination consists of individual components, these are to be mixed with one another. After mixing the sample (for example substrate solution, enzyme solution, blood, serum, plasma or urine) with an aliquot portion of the reagent mixture, the resulting colour is measured on a photometer and the particular concentration or substrate concentration is calculated via the molar extinction coefficients and the volumes

of reagent and sample added. Both kinetic and end point measurements are possible.

The compounds of the general formula (I) can also be impregnated, together with the reagent or reagents necessary for the particular parameter detection or other enzymes, the buffer system, if appropriate wetting agents and activators as well as other auxiliaries, onto absorbent reagent carriers, such as paper, nonwoven materials and the like. For this, one or more impregnating solutions can be prepared in the form of aqueous or organic or mixed solutions, depending on how the reagents or auxiliaries dissolve. Absorbent or swellable carriers, preferably filter paper or absorbent nonwoven glass or plastic, are impregnated or sprayed with these solutions. The system is then dried. The reagent carriers thus prepared can be used either as rapid diagnostics for direct determination of the contents of liquids (for example in body fluids, such as blood, urine or saliva, or in foodstuffs, for example fruit juices, milk or the like). The liquid is thereby applied directly to the reagent carrier or this is immersed briefly in the liquid. Semi-quantitative determination is possible by allocating the resulting colour a comparison colour. Quantitative evaluation can be carried out by reflectance photometry.

It is also possible to introduce the compounds of the general formula (I) into carrier matrices which have been prepared from casting solutions. Cellulose, cellulose derivatives, gelatine, gelatine derivatives or even plastics, such as polyurethanes and acrylamide, may be mentioned as examples here. It is advantageous if the compounds of the general formula (I) and if appropriate the other reagents required are added directly to the casting solution, whereupon it is possible for the test device, consisting of carrier and reagents, to be produced in one operation.

A reagent solution with which substrates or enzymes can be determined in the cell on the photometer as described above can be prepared by eluting the above-mentioned reagents from the absorbent carrier with water or a buffer or serum.

Suitable buffers for the test agents mentioned are phosphate, citrate, borate and GOOD buffers with alkali metal or ammonium counterions. However, other systems can also be used. pH values which are to be aimed for are 6 to 10, in particular 6.5 to 7.5.

Wetting agents are, in particular, anionic and cationic wetting agents which undergo ionic interactions with the compounds according to the invention. However, non-ionic wetting agents which activate the enzymes can also be used. Sodium lauryl-sulphate, dioctyl sodium sulphosuccinate and alkylaryl polyether-alcohols are preferred.

The effectors to be employed are those known for the particular enzymatic reaction.

Other auxiliaries which may be appropriate are customary thickeners, solubilizing agents, emulsifiers, optical brighteners, contrast agents and the like, such as are known in corresponding tests with other chromogens.

Example 1

130 g of the sodium salt of 2-chloro-5-nitrobenzenesulphonic acid are added to a solution of 120 g of sodium sulphide.$9H_2O$ in 750 ml of water. The mixture is stirred at room temperature for 10 hours and the solution is acidified. The hydrogen sulphide formed is removed with the aid of nitrogen. A pH value of 10 is then established with sodium hydroxide solution and the oxidation is carried out with 125 g of iodine to give the disulphide. During the addition of iodine, the pH value must be continuously adjusted with sodium hydroxide solution. When the oxidation has ended, a pH value of 7.5 is established and the following compound is salted out with 20% of sodium chloride.

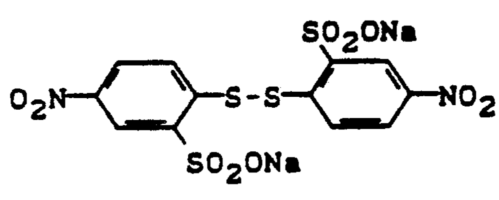

Example 2

101 g of 3,4-dinitro-chlorobenzene are heated under reflux in a solution of 500 ml of water and 126 g of sodium sulphite for 30 hours. The mixture is allowed to cool and the yellow precipitate which has separated out, of the following formula, is recrystallized from water.

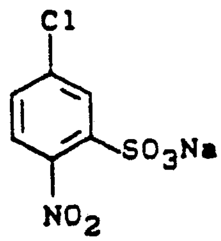

25.9 g of the sodium 3-chloro-6-nitro-benzenesulphonate prepared above are added to a solution of 25.0 of sodium sulphide.$9H_2O$ in 250 ml of water at room temperature. After a reaction time of 5 hours (thin layer chromatography control), the solution is acidified, the hydrogen sulphide which has formed is removed with nitrogen and the solution is then clarified over active charcoal. The yellow filtrate containing the mercapto compound of the following structure

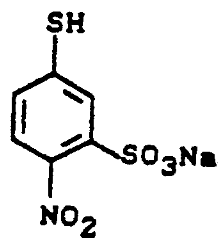

is then brought to pH 10 with sodium hydroxide solution and oxidized with 25 g of iodine. The pH value is maintained during the oxidation by addition of sodium hydroxide solution. The disulphide formed is salted out with sodium chloride and recrystallized from water. It is characterized by the following $^{13}C$-NMR spectrum.

$^{13}C$ shifts in DMSO-$D_6$

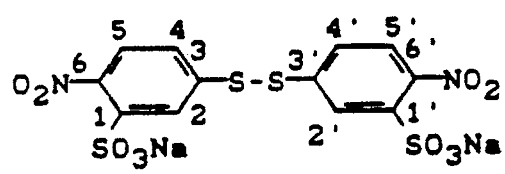

| C atoms | δ [ppm] |
|---|---|
| C-1/C-1' | 138.300 |
| C-2/C-2' | 126.478 |
| C-3/C-3' | 140.481 |
| C-4/C-4' | 127.248 |
| C-5/C-5' | 123.948 |
| C-6/C-6' | 146.616 |

<u>Example 3</u>

50.0 g of N-methyl-piperazine are dissolved in 300 ml of chloroform. After addition of 69 ml of triethylamine, 128 g of 2-chloro-5-nitrobenzenesulphonyl chloride are introduced at room temperature. After 5 hours, the mixture is poured onto ice and the sulphonamide formed, of the following formula, is extracted with chloroform.

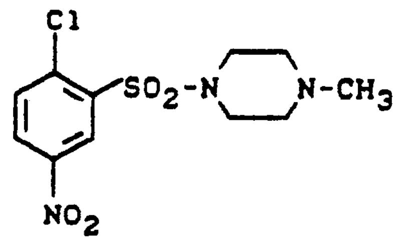

A freshly prepared sodium disulphide solution obtained from 48.0 g of sodium sulphide.$9H_2O$ in 250 ml of ethanol with 6.4 g of sulphur is added dropwise to a solution of 63.9 g of the nitro compound prepared above in 300 ml of ethanol. The mixture is boiled under a reflux condenser for 2 hours and is then poured into 1 l of ice-water. The pale yellow product which has precipitated is filtered off with suction and crystallized from ethanol to give a melting point of 205° C.

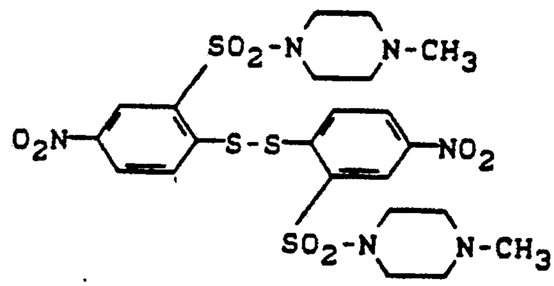

Example 4

25.9 g of the sodium salt of 3-chloro-6-nitrobenzenesulphonic acid are added in portions to a mixture of 50 ml of phosphorus oxychloride and 25 g of phosphorus pentachloride at 80° C. After 2 hours, the evolution of HCl has subsided. The solution is concentrated on a rotary evaporator, the residue is taken up in toluene, the toluene phase is washed with ice-water and the extract is dried and evaporated again on a rotary evaporator. The sulphochloride of the following formula remains as a pale crystalline mass.

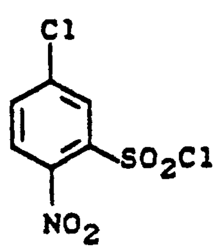

25.6 g of 3-chloro-6-nitrobenzenesulphonyl chloride, dissolved in 100 ml of chloroform, are added dropwise to 250 ml of chloroform containing a mixture of 10.0 g of N-methylpiperazine and 11 ml of triethylamine at room temperature. When the reaction has ended, the mixture is poured onto ice and extracted with chloroform and the following sulphonamide is thus isolated.

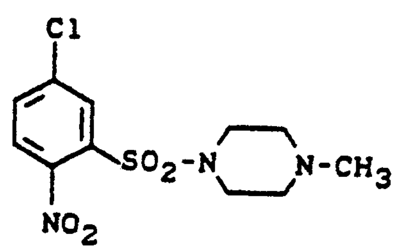

31.95 g of the sulphonamide prepared above are then added to a suspension of 25.0 g of sodium sulphide. $9H_2O$ in 100 ml of dimethylformamide at room temperature. After 1 hour, the chloride/sulphide replacement reaction has ended. The reaction mixture is poured onto ice, acidified and filtered with suction. 27.3 g of the following mercapto compound are obtained.

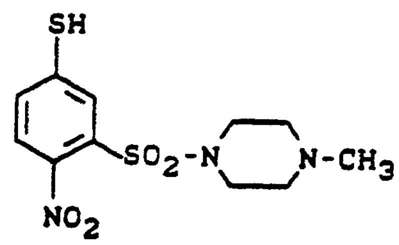

15.85 g of the mercapto compound prepared above are dissolved in a mixture of 200 ml of ethanol and 50 ml of triethylamine and 12.5 g of iodine are then added in portions. The end of the reaction is indicated by the change in colour from red-brown to yellow. The reaction product is poured onto ice and the pale yellow precipitate is filtered off with suction. The disulphide of the following structure, recrystallized from chloroform/ethanol, melts from 173 ° C (decomposition)

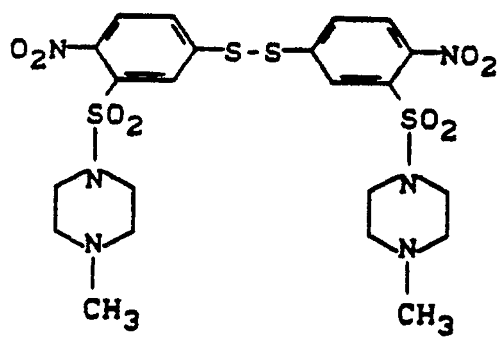

Example 5

58.0 g of sodium phenolate are dissolved in 300 ml of water. 128 g of 4-chloro-5-nitrobenzenesulphonyl chloride are added in portions at room temperature. The mixture is stirred overnight and the product of the following formula which has precipitated is filtered off with suction.

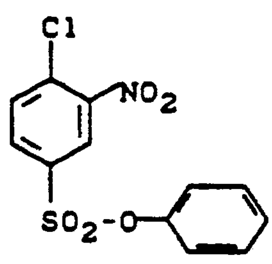

25.0 g of sodium sulphide.$9H_2O$ are suspended in 100 ml of dimethylformamide and 91.3 g of phenyl 4-chloro-5-nitro-benzenesulphonate are then added at room temperature. After 1 hour, the mixture is poured onto ice and acidified and the mercapto compound is filtered off with suction.

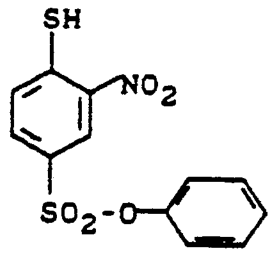

15.55 g of the mercapto compound prepared above are dissolved in 200 ml of ethanol and 50 ml of triethylamine. The mercapto compound is converted into the disulphide by addition of 12.0 g of iodine. The compound of the following formula melts at 148 - 151 °C.

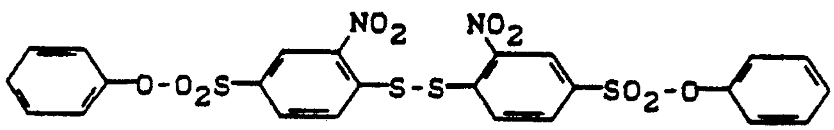

Example 6

A solution of 19.0 g of 4-nitrobenzenesulphenyl chloride in 200 ml of chloroform is added dropwise to a solution of 27.9 g of phenyl 4-mercapto-5-nitro-benzenesulphonate in 500 ml of chloroform at 0 °C. Removal of the HCl gas formed is taken care of with a dry stream of nitrogen. When the reaction has ended, the mixture is evaporated on a rotary evaporator and the residue is recrystallized from ethanol. 39.8 g of the following disulphide are obtained.

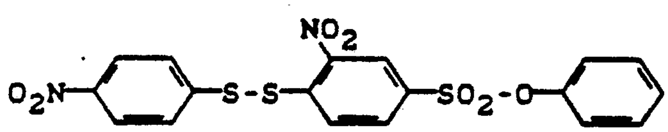

Example 7

3.04 g of the 3-mercapto-6-nitrobenzenesulphonic acid morpholide prepared according to Example 4 are dissolved in 50 ml of methylene chloride. A solution of 2.0 g of 4-nitrobenzenesulphenyl chloride in 30 ml of methylene chloride are added dropwise, while cooling with ice. The HCl gas formed is removed with a stream of nitrogen. When the reaction has ended, the methylene chloride is stripped off in vacuo and the residue is recrystallized from acetonitrile. 4.5 g of the following compound are obtained.

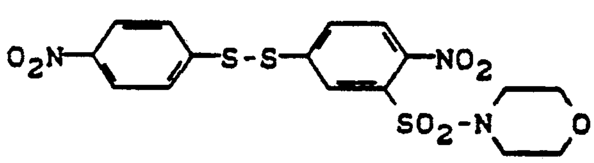

The following disulphides have been prepared by the methods described in Examples 1 to 7.

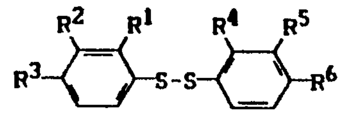

| Example | $R^1$ | $R^2$ | $R^3$ | $R^4$ | $R^5$ | $R^6$ |
|---|---|---|---|---|---|---|
| 8 | $NO_2$ | H | $SO_3H$ | $NO_2$ | H | $SO_3H$ |
| 9 | $NO_2$ | H | 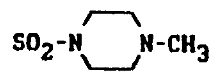 | $NO_2$ | H | 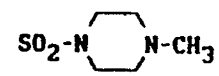 |
| 10 | $NO_2$ | H | $SO_2NHCH_2CH_2SO_3H$ | $NO_2$ | H | $SO_2NHCH_2CH_2SO_3H$ |
| 11 | $NO_2$ | H | 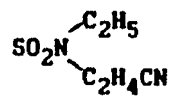 | $NO_2$ | H | 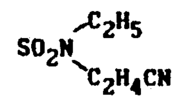 |
| 12 | H | 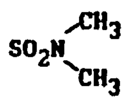 | $NO_2$ | H | 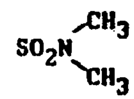 | $NO_2$ |
| 13 | H | $SO_2NHCH_2COOH$ | $NO_2$ | H | $SO_2NHCH_2COOH$ | $NO_2$ |
| 14 | H | 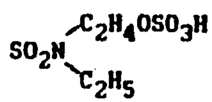 | $NO_2$ | H | 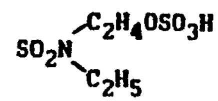 | $NO_2$ |

| Example | $R^1$ | $R^2$ | $R^3$ | $R^4$ | $R^5$ | $R^6$ |
|---|---|---|---|---|---|---|
| 15 | H | 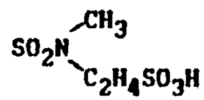 | $NO_2$ | H | 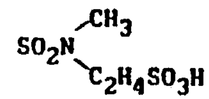 | $NO_2$ |
| 16 | 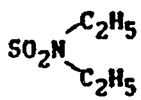 | H | $NO_2$ | 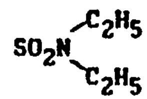 | H | $NO_2$ |
| 17 | 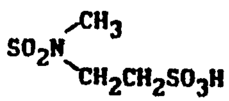 | H | $NO_2$ | 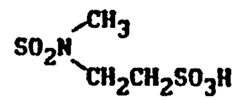 | H | $NO_2$ |
| 18 | 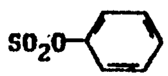 | H | $NO_2$ | 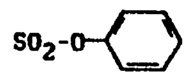 | H | $NO_2$ |
| 19 | H | 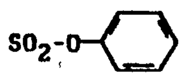 | $NO_2$ | H | 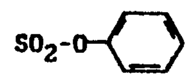 | $NO_2$ |
| 20 | $NO_2$ | H | $NO_2$ | $NO_2$ | H | 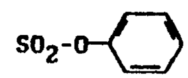 |

| Example | $R^1$ | $R^2$ | $R^3$ | $R^4$ | $R^5$ | $R^6$ |
|---|---|---|---|---|---|---|
| 21 | $NO_2$ | H | H | H | $SO_2N(C_2H_5)(C_2H_4CN)$ | $NO_2$ |
| 22 | H | H | $NO_2$ | $NO_2$ | H | $SO_2N(C_2H_4Cl)(C_2H_5)$ |
| 23 | $NO_2$ | H | $SO_2N(C_2H_4OCH_3)(C_2H_5)$ | $NO_2$ | H | $SO_2N(C_2H_4OCH_3)(C_2H_5)$ |
| 24 | H | $SO_2$-O-$C_4H_9$ | $NO_2$ | H | $SO_2$-O-$C_4H_9$ | $NO_2$ |
| 25 | H | $SO_2N(C_2H_4OH)(C_2H_5)$ | $NO_2$ | H | $SO_2N(C_2H_4OH)(C_2H_5)$ | $NO_2$ |
| 26 | H | $SO_2N(C_2H_4OSO_3H)(C_2H_4OSO_3H)$ | $NO_2$ | H | $SO_2N(C_2H_4OSO_3H)(C_2H_4OSO_3H)$ | $NO_2$ |
| 27 | H | $SO_2NHC_2H_4SO_3H$ | $NO_2$ | H | $SO_2NHC_2H_4SO_3H$ | $NO_2$ |

| Example | $R^1$ | $R^2$ | $R^3$ | $R^4$ | $R^5$ | $R^6$ |
|---|---|---|---|---|---|---|
| 28 | H | $SO_2NHC_2H_4SO_3H$ | $NO_2$ | $NO_2$ | H | $NO_2$ |
| 29 | H | H | $NO_2$ | H | $SO_2NHC_2H_4SO_3H$ | $NO_2$ |
| 30 | $NO_2$ | H | H | 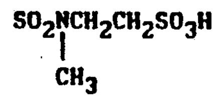 | H | $NO_2$ |
| 31 | 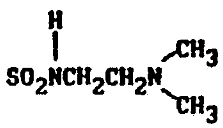 | H | $NO_2$ | 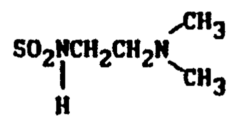 | H | $NO_2$ |
| 32 | $NO_2$ | H | 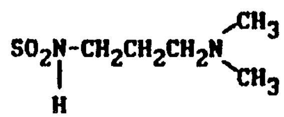 | $NO_2$ | H | 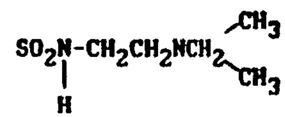 |
| 33 | H | 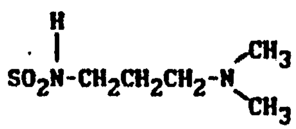 | $NO_2$ | H | 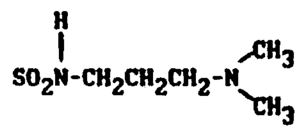 | $NO_2$ |
| 34 | 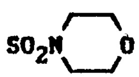 | H | $NO_2$ | 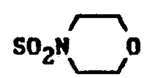 | H | $NO_2$ |

| Example | $R^1$ | $R^2$ | $R^3$ | $R^4$ | $R^5$ | $R^6$ |
|---|---|---|---|---|---|---|
| 35 | $SO_2N(C_4H_9)_2$ | H | $NO_2$ | $SO_2N(C_4H_9)_2$ | H | $NO_2$ |
| 36 | $NO_2$ | H | $SO_2N(C_2H_5)_2$ | $NO_2$ | H | $SO_2N(C_2H_5)_2$ |

Example 37

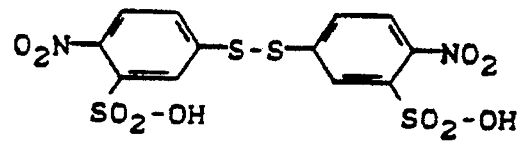

Compound from Example 2

To test the compound from Example 2 as an indicator for NADH in the test system described, the compound was dissolved in water to give a 20 mM solution. The following reagent constituents were pipetted into a cell together: 1,810 µl of buffer (0.1 M/l of Tris, pH 7), 100 µl of the compound from Example 2 (20 mmol/l in $H_2O$), 50 µl of lipoic amide (50 mmol/l) and 20 µl of lipoic amide dehydrogenase (1,200 kU/l).

After measurement of the reagent blank value, the reaction was started by addition of 20 µl of NADH. The extinction maximum measured is at 400 nm. Kinetic investigations showed a stable end point (change in extinction within 20 minutes <1%) after a reaction time of only 1 minute.

To test for functionality and linearity, NADH concentrations in the range from 1 to 10 mmol/l were measured in the test mixture. The extinction differences measured at 400 nm are summarized in Table 1.

Table 1:

| NADH (mmol/L) | Compound from Example 37 pH 7 $E_1$ | $E_2$ | $\Delta E_{400}$ nm | Comparison DTNB (Ellman) pH 7.5 $E_1$ | $E_2$ | $\Delta E_{430}$ nm |
|---|---|---|---|---|---|---|
| 1 | 0.391 | 0.529 | 0.138 | 0.159 | 0.348 | 0.189 |
| 2 | 0.382 | 0.663 | 0.281 | 0.160 | 0.553 | 0.393 |
| 3 | 0.384 | 0.813 | 0.429 | 0.158 | 0.755 | 0.597 |
| 4 | 0.381 | 0.957 | 0.576 | 0.159 | 0.958 | 0.799 |
| 5 | 0.382 | 1.104 | 0.722 | 0.159 | 1.153 | 0.994 |
| 6 | 0.382 | 1.244 | 0.862 | 0.158 | 1.359 | 1.201 |
| 7 | 0.382 | 1.389 | 1.007 | 0.157 | 1.550 | 1.393 |
| 8 | 0.383 | 1.536 | 1.153 | 0.157 | 1.765 | 1.608 |
| 9 | 0.383 | 1.676 | 1.293 | 0.158 | 1.956 | 1.798 |
| 10 | 0.384 | 1.792 | 1.498 | 0.160 | 2.104 | 1.944 |

Table 2 shows the results of the test for interference by ascorbic acid. For this test, 20 µl of an NADH solution (5 mmol/l) and also 20 µl of an ascorbic acid solution of 1 mmol/l are added to the above mixture and the resulting extinctions are measured.

Table 2:

| | $E_1$ | $E_2$ | $\Delta E_{400}$ nm |
|---|---|---|---|
| without ascorbic acid | | | |
| | 0.379 | 1.089 | 0.710 |
| | 0.386 | 1.097 | 0.711 |
| | 0.383 | 1.093 | 0.710 x = 0.710 |
| with ascorbic acid | | | |
| | 0.383 | 1.089 | 0.706 |
| | 0.381 | 1.088 | 0.707 |
| | 0.384 | 1.087 | 0.703 x = 0.705 |

The values measured in the presence of ascorbic acid are on average 0.7% above the values measured in the absence of ascorbic acid. The present test is not influenced by ascorbic acid.

Example 38

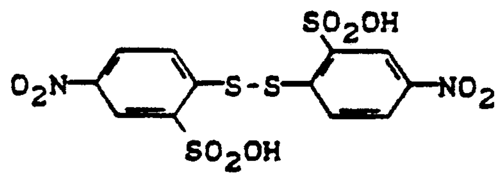

Testing of the compound from Example 1. The same reaction conditions as in Example 37 were chosen. However, 0.1 M Tris pH 9 was chosen as the buffer, since a faster reaction (end point within 1 minute) was achieved with this substance at pH 9 than at pH 7.

The absorption maximum measured is at 425 nm. The coloration is stable within the period measured (20 minutes).

The test for ascorbic acid interference was carried out analogously to Example 37. Table 3 shows the results.

Table 3:

| | $E_1$ | $E_2$ | $\Delta E_{425}$ nm |
|---|---|---|---|
| without ascorbic acid | | | |
| | 0.145 | 1.410 | 1.265 |
| | 0.142 | 1.411 | 1.269 |
| | 0.143 | 1.410 | 1.267 x = 1.267 |
| with ascorbic acid | | | |
| | 0.144 | 1.424 | 1.280 |
| | 0.147 | 1.417 | 1.270 |
| | 0.150 | 1.407 | 1.257 x = 1.269 |

In the presence of ascorbic acid, the values are on average 0.2% higher. Ascorbic acid does not interfere with the present test.

Table 4 shows the function and linearity testing after addition of NADH concentrations in the range from 1 to 10 mmol/l.

Table 4

| NADH (mmol/L) | Compound from Example 38 pH 9 $E_1$ | $E_2$ | $\Delta E_{425}$ nm | Comparison: DTNB (Ellmann) pH 7.5 $E_1$ | $E_2$ | $\Delta E_{430}$ nm |
|---|---|---|---|---|---|---|
| 1 | 0.154 | 0.404 | 0.250 | 0.159 | 0.348 | 0.189 |
| 2 | 0.150 | 0.663 | 0.513 | 0.160 | 0.553 | 0.393 |
| 3 | 0.147 | 0.914 | 0.767 | 0.158 | 0.755 | 0.597 |
| 4 | 0.148 | 1.185 | 1.037 | 0.159 | 0.958 | 0.799 |
| 5 | 0.149 | 1.452 | 1.303 | 0.159 | 1.153 | 0.994 |
| 6 | 0.148 | 1.696 | 1.548 | 0.158 | 1.359 | 1.201 |
| 7 | 0.151 | 1.970 | 1.819 | 0.157 | 1.550 | 1.393 |
| 8 | 0.158 | 2.220 | 2.062 | 0.157 | 1.765 | 1.608 |
| 9 | 0.158 | 2.420 | 2.270 | 0.158 | 1.956 | 1.798 |
| 10 | 0.146 | 2.655 | 2.509 | 0.160 | 2.104 | 1.944 |

Example 39

To determine the extinction maxima, further compounds were dissolved in dimethylformamide and in each case 2 ml of 0.1 M/l Tris buffer pH 9 and 10 µl of mercaptoethanol were added to 50 µl of the solution. The extinction maxima are shown in Table 5.

Table 5

| | Extinction maximum |
|---|---|
| Example 34 | 420 nm |
| Example 36 | 434 nm |
| Example 35 | 464 nm |
| Example 16 | 445 nm |

## Claims

1. Test agents for the detection of thiol groups, containing as the indicator for the thiol groups compounds of the general formula I

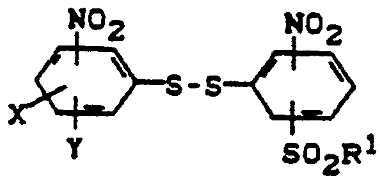

I

wherein
X represents hydrogen or $-SO_2R^1$,
Y represents hydrogen or $-NO_2$,
$R^1$ represents $-O-R^2$ or

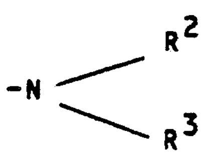

and
$R^2$ and $R^3$ independently of one another represent hydrogen or $C_1$-$C_4$-alkyl, which can be substituted by halogen, -COOH, cyano, $C_1$-$C_4$-alkoxy, hydroxyl, $-OSO_3H$, $-SO_3H$ or amino, it being possible for the amino group in turn to be substituted by $C_1$-$C_4$-alkyl which is optionally substituted by halogen, hydroxyl, cyano, $-OSO_3H$ or $-SO_3H$, or by phenyl or benzyl groups, or $R^2$ and $R^3$, together with the N atom, represent a pyrrolidine, pyrazoline, piperidine, piperazine or morpholine ring, which can be substituted by $C_1$- to $C_4$-alkyl or phenyl.

2. Agents according to Claims 1 containing compounds of the general formula IV

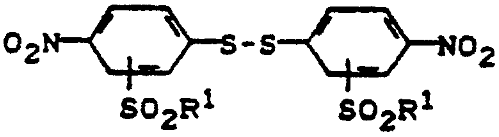

IV

wherein
$R^1$ has the meaning given in Claim 1.

3. Agents according to Claims 1 to 2, containing compounds of the general formula V

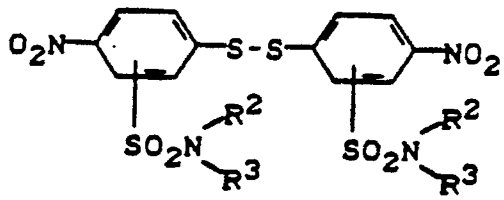

V

wherein
$R^2$ and $R^3$ have the meaning given in Claim 1.

**4.** Agents according to Claims 1 to 3, additionally containing one or more substances from the group comprising enzymes, substrates, coenzymes, effectors, antigens, antibodies, buffers, wetting agents and stabilizers.

**5.** Use of the agent according to Claims 1 to 4 for the detection of thiol groups in a sample.

**6.** Compounds of the general formula I

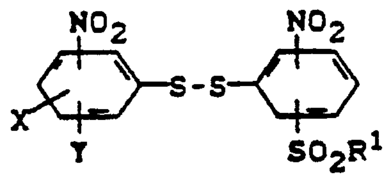

I

wherein
X represents hydrogen or $-SO_2R^1$,
Y represents hydrogen or $-NO_2$,
$R^1$ represents $-O-R^2$ or

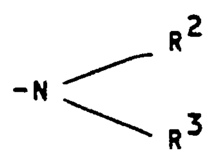

$R^3$ represents hydrogen or $C_1$-$C_4$-alkyl, which can be substituted by halogen, -COOH, cyano, $C_1$-$C_4$-alkoxy, hydroxyl, $-OSO_3H$, $-SO_3H$ or amino, it being possible for the amino group in turn to be substituted by $C_1$-$C_4$-alkyl which is optionally substituted by halogen, hydroxyl, cyano, $-OSO_3H$ or $-SO_3H$, or by phenyl or benzyl groups,
and
$R^2$ has the same meaning as $R^3$, apart from hydrogen, or
$R^2$ and $R^3$ together with the N atom, represent a pyrrolidine, pyrazoline, piperidine, piperazine or morpholine ring, which can be substituted by $C_1$- to $C_4$-alkyl or phenyl.

**7.** Compounds of the general formula IV

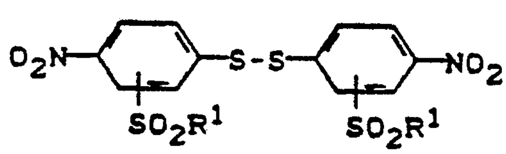

IV

wherein
$R^1$ has the meaning given in Claim 6.

**8.** Compounds of the general formula V

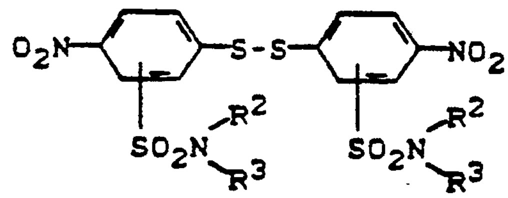

V

wherein
$R^2$ and $R^3$ have the meaning given in Claim 6.

**9.** Compounds of the general formula VI

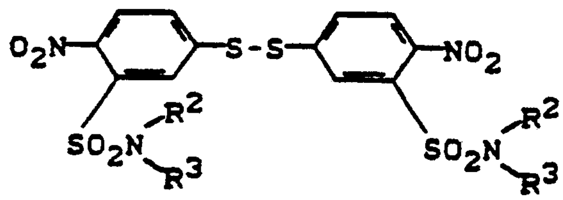

VI

wherein
$R^2$ and $R^3$ have the meaning described in Claim 6.

**10.** Process for the preparation of compounds of the general formula I

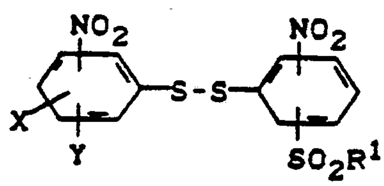

I

wherein
X represents hydrogen or $-SO_2R^1$,
Y represents hydrogen or $-NO_2$,
$R^1$ represents $-O-R^2$ or

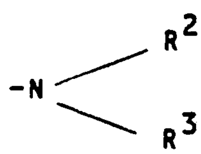

$R^3$ represents hydrogen or $C_1$-$C_4$-alkyl, which can be substituted by halogen, -COOH, cyano, $C_1$-$C_4$-alkoxy, hydroxyl, $-OSO_3H$, $-SO_3H$ or amino, it being possible for the amino group in turn to be substituted by $C_1$-$C_4$-alkyl which is optionally substituted by halogen, hydroxyl, cyano, $-OSO_3H$ or $-SO_3H$, or by phenyl or benzyl groups,
and
$R^2$ has the same meaning as $R^3$, apart from hydrogen, or
$R^2$ and $R^3$ together with the N atom, represent a pyrrolidine, pyrazoline, piperidine, piperazine or morpholine ring, which can be substituted by $C_1$- to $C_4$-alkyl or phenyl,
characterized in that a chloro-nitro-benzenesulphonyl chloride of the formula

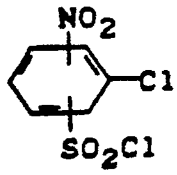

is converted with an amine or alcoholate of the fcrmula H-R[1], wherein R[1] has the abovementioned meaning, into the corresponding chloro-nitro-sulphonic acid derivative and a chloride/sulphide replacement reaction is then carried out in the presence of $Na_2S$, followed by oxidation, a symmetric disulphide being obtained, or in that a mercapto-nitro-benzene-sulphonic acid derivative obtained by chloride/sulphide replacement reaction, of the formula

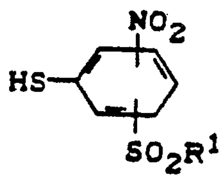

wherein R[1] has the abovementioned meaning, is reacted with a substituted phenylsulphenyl chloride of the formula

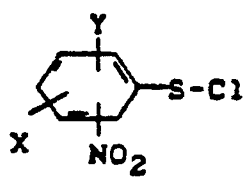

wherein X and Y have the abovementioned meaning, whereupon an asymmetric disulphide is obtained.

**Revendications**

1. Réactifs de tests pour la détection de groupes thiols contenant comme indicateur pour les groupes thiols des composés de formule générale I

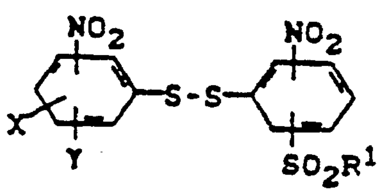

I

dans laquelle
X représente l'hydrogène ou $-SO_2R^1$,
Y représente l'hydrogène ou $-NO_2$,
$R^1$ représente $-O-R^2$ ou

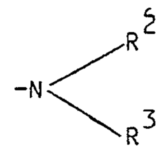

et

$R^2$ et $R^3$ représentent indépendamment l'hydrogène ou alkyle en $C_1$-$C_4$, qui peut être substitué par halogène, -COOH, cyano, alcoxy en $C_1$-$C_4$, hydroxyle, $-OSO_3H$, $-SO_3H$ ou amino, le groupe amino pouvant à son tour être substitué par alkyle en $C_1$-$C_4$ qui est facultativement substitué par halogène, hydroxyle, cyano, $-OSO_3H$ ou $-SO_3H$, ou par des groupes phényle ou benzyle, ou bien $R^2$ et $R^3$, pris ensemble avec l'atome d'azote, représentent un noyau pyrrolidine, pyrazoline, pipéridine, pipérazine ou morpholine, qui peut être substitué par alkyle en $C_1$-$C_4$ ou phényle.

**2.** Réactifs selon la revendication 1, contenant des composés de formule générale IV

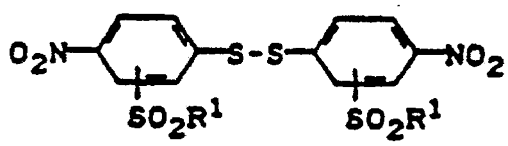

IV

dans laquelle
$R^1$ a la signification indiquée à la revendication 1.

**3.** Réactifs selon les revendications 1 à 2, contenant des composés de formule générale V

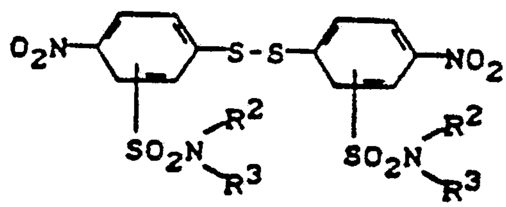

V

dans laquelle
$R^2$ et $R^3$ ont les significations indiquées à la revendication 1.

**4.** Réactifs selon les revendications 1 à 3, contenant en outre une ou plusieurs substances choisies parmi les enzymes, les substrats, les coenzymes, les effecteurs, les antigènes, les anti-corps, les tampons, les agents mouillants et les stabilisants.

**5.** Utilisation du réactif selon les revendications 1 à 4 pour la détection de groupes thiols dans un échantillon.

**6.** Composés de formule générale I

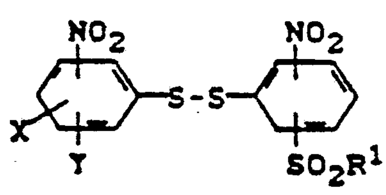

I

dans laquelle
X représente l'hydrogène ou $-SO_2R^1$,
Y représente l'hydrogène ou $-NO_2$,
$R^1$ représente $-O-R^2$ ou

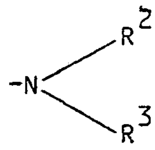

$R^3$ représente l'hydrogène ou alkyle en $C_1$-$C_4$, qui peut être substitué par halogène, -COOH, cyano, alcoxy en $C_1$-$C_4$, hydroxyle, $-OSO_3H$, $-SO_3H$ ou amino, le groupe amino pouvant à son tour être substitué par alkyle en $C_1$-$C_4$ qui est facultativement substitué par halogène, hydroxyle, cyano, $-OSO_3H$ ou $-SO_3H$, ou par des groupes phényle ou benzyle,
et
$R^2$ a la même signification que $R^3$, sauf l'hydrogène, ou bien
$R^2$ et $R^3$, pris ensemble avec l'atome d'azote, représentent un noyau pyrrolidine, pyrazoline, pipéridine, pipérazine ou morpholine, qui peut être substitué par alkyle en $C_1$-$C_4$ ou phényle.

**7.** Composés de formule générale IV

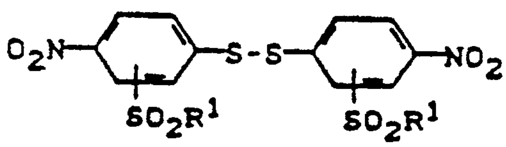

IV

dans laquelle
$R^1$ a la signification indiquée à la revendication 6.

**8.** Composés de formule générale V

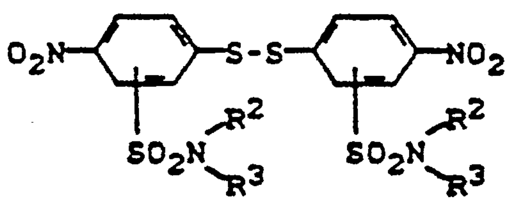

V

dans laquelle
$R^2$ et $R^3$ ont les significations indiquées à la revendication 6.

**9.** Composés de formule générale VI

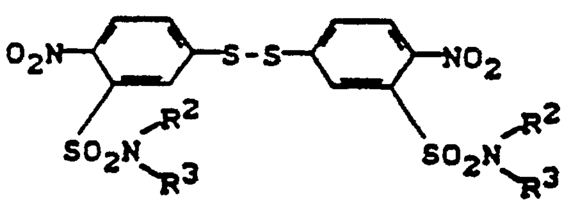

VI

dans laquelle
$R^2$ et $R^3$ ont les significations indiquées à la revendication 6.

**10.** Procédé pour la préparation de composés de formule générale I

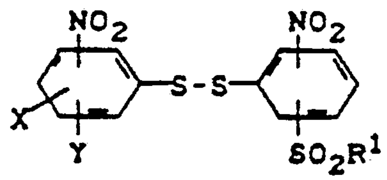

I

dans laquelle
X représente l'hydrogène ou $-SO_2R^1$,
Y représente l'hydrogène ou $-NO_2$,
$R^1$ représente $-O-R^2$ ou

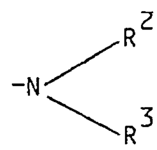

$R^3$ représente l'hydrogène ou alkyle en $C_1$-$C_4$, qui peut être substitué par halogène, -COOH, cyano, alcoxy en $C_1$-$C_4$, hydroxyle, $-OSO_3H$, $-SO_3H$ ou amino, le groupe amino pouvant à son tour être substitué par alkyle en $C_1$-$C_4$ qui est facultativement substitué par halogène, hydroxyle, cyano, $-OSO_3H$ ou $-SO_3H$, ou par des groupes phényle ou benzyle,
et
$R^2$ a la même signification que $R^3$, sauf l'hydrogène, ou bien
$R^2$ et $R^3$, pris ensemble avec l'atome d'azote, représentent un noyau pyrrolidine, pyrazoline, pipéridine, pipérazine ou morpholine, qui peut être substitué par alkyle en $C_1$-$C_4$ ou phényle,
caractérisé en ce qu'un chlorure de chloro-nitro-benzènesulfonyle de formule

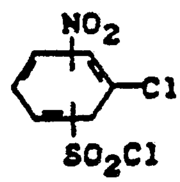

est transformé en le dérivé correspondant d'acide chloro-nitro-sulfonique par réaction avec une amine ou un alcoolate de formule $H-R^1$,
dans laquelle
$R^1$ a la signification citée ci-dessus et une réaction de remplacement chlorure/sulfure est ensuite mise en oeuvre en présence de $Na_2S$, suivie d'oxydation, avec obtention d'un disulfure symétrique, ou en ce qu'un dérivé d'acide mercapto-nitro-benzènesulfonique obtenu par une réaction de remplacement chlorure/ sulfure, de formule

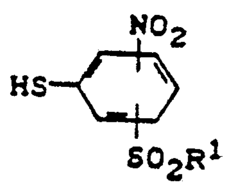

dans laquelle
$R^1$ a la signification citée ci-dessus,
est mis à réagir avec un chlorure de phénylsulfényle substitué de formule

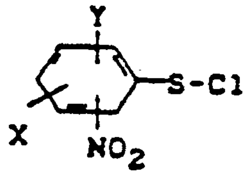

dans laquelle

X et Y ont les significations indiquées ci-dessus, avec obtention d'un disulfure asymétrique.

**Patentansprüche**

1. Testagenzien zum Nachweis von Thiolgruppen, die als Indikator für die Thiolgruppen Verbindungen der allgemeinen Formel (1)

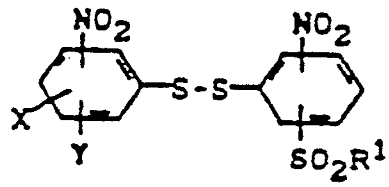

(I)

enthalten, in der

X Wasserstoff oder $SO_2R^1$ ist;

Y Wasserstoff oder $-NO_2$ ist;

$R^1$ -O-$R^2$ oder

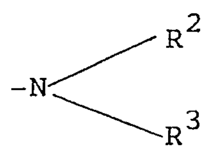

ist; und

$R^2$ und $R^3$ unabhängig voneinander folgende Bedeutungen haben: Wasserstoff oder $C_{1-4}$-Alkyl, welches substituiert sein kann durch Halogen, -COOH, Cyan, $C_{1-4}$-Alkoxy, Hydroxyl, $-OSO_3H$, $-SO_3H$ oder Amino, wobei es möglich ist, dass diese Aminogruppe durch $C_{1-4}$-Alkyl substituiert ist, welches beliebig substituiert ist durch Halogen, Hydroxyl, Cyan, $-OSO_3H$ oder $-SO_3H$, oder durch Phenyl- oder Benzylgruppen, oder $R^2$ und $R^3$ zusammen mit dem Stickstoffatom einen Pyrrolidin-, Pyrazolin-Piperidin, Piperazin- oder Morpholinring darstellen, welcher durch $C_{1-4}$-Alkyl oder Phenyl substituiert sein kann.

2. Agenzien nach Anspruch 1, enthaltend Verbindungen der allgemeinen Formel (IV)

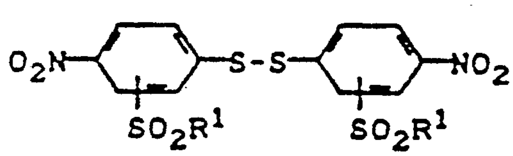

(IV)

in der $R^1$ die in Anspruch 1 angegebene Bedeutung hat.

3. Agenzien nach Ansprüchen 1 bis 2, enthaltend Verbindungen der allgemeinen Formel (V)

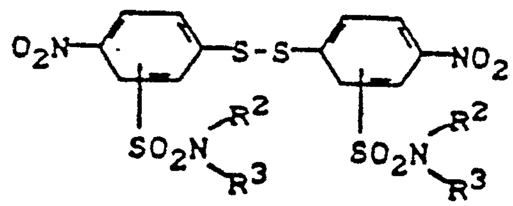

(V)

in der $R^2$ und $R^3$ die in Anspruch 1 angegebenen Bedeutungen haben.

4. Agenzien nach Ansprüchen 1 bis 3, die zusätzlich eine oder mehrere Substanzen aus der Gruppe, die Enzyme, Substrate, Coenzyme, Effektoren, Antigene, Antikörper, Puffer, Benetzungsmittel und Stabilisatoren umfasst, enthalten.

5. Verwendung des Agenses nach Ansprüchen 1 bis 4 zum Nachweis von Thiolgruppen in einer Probe.

6. Verbindungen der allgemeinen Formel (I)

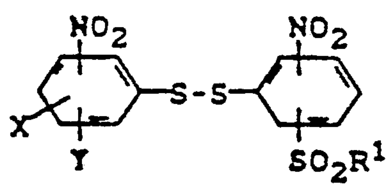

(I)

in der X Wasserstoff oder $-SO_2R^1$ ist,

Y Wasserstoff oder $-NO_2$ ist;

$R^1$ $-O-R^2$ oder

$$-N\begin{matrix} \diagup R^2 \\ \diagdown R^3 \end{matrix}$$

ist; und

$R^3$ folgende Bedeutungen hat: Wasserstoff oder $C_{1-4}$-Alkyl, welches substituiert sein kann durch Halogen, -COOH, Cyan, $C_{1-4}$-Alkoxy, Hydroxyl, $-OSO_3H$, $-SO_3H$ oder Amino, wobei es möglich ist, dass diese Aminogruppe durch $C_{1-4}$-Alkyl substituiert ist, welches beliebig substituiert ist durch Halogen, Hydroxyl, Cyan, $-OSO_3H$ oder $-SO_3H$, oder durch Phenyl- oder Benzylgruppen, und wobei $R^2$ die gleiche Bedeutung wie $R^3$ hat, ausser Wasserstoff, oder wobei $R^2$ und $R^3$ zusammen mit dem N-Atom einen Pyrrolidin-, Pyrazolin-, Piperidin-, Piperazin- oder Morpholinring darstellen, welcher durch $C_{1-4}$-Alkyl oder Phenyl substituiert sein kann.

7. Verbindungen der allgemeinen Formel (IV)

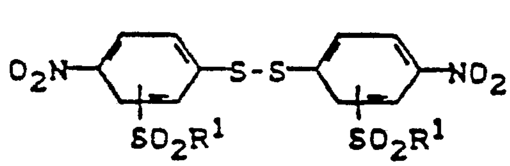

(IV)

in der $R^1$ die in Anspruch 6 angegebene Bedeutung hat.

**8.** Verbindungen der allgemeinen Formel (V)

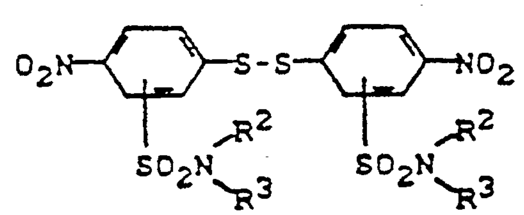

(V)

in der $R^2$ und $R^3$ die in Anspruch 6 angegebenen Bedeutungen haben.

**9.** Verbindungen der allgemeinen Formel (VI)

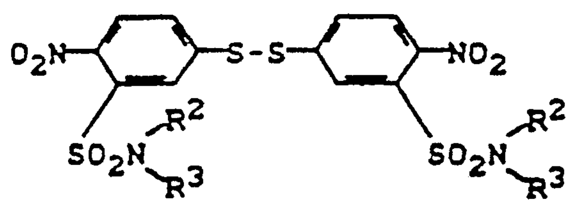

(VI)

in der $R^2$ und $R^3$ die in Anspruch 6 beschriebenen Bedeutungen haben.

**10.** Verfahren zur Herstellung von Verbindungen der allgemeinen Formel (I)

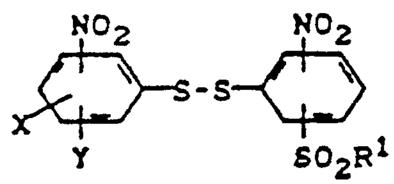

(I)

in der

X Wasserstoff oder $-SO_2R^1$ ist;

Y Wasserstoff oder $-NO_2$ ist;

$R^1$ $-O-R^2$ oder

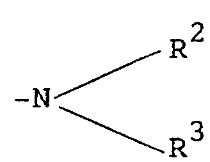

ist;

$R^3$ folgende Bedeutungen hat: Wasserstoff oder $C_{1-4}$-Alkyl, welches substituiert sein kann durch Halogen, -COOH, Cyan, $C_{1-4}$-Alkoxy, Hydroxyl, $-OSO_3H$, $-SO_3H$ oder Amino, wobei es möglich ist, dass diese Aminogruppe durch $C_{1-4}$-Alkyl substituiert ist, welches beliebig substituiert ist durch Halogen, Hydroxyl, Cyan, $-OSO_3H$ oder $-SO_3H$, oder durch Phenyl- oder Benzylgruppen, und wobei $R^2$ die gleiche Bedeutung wie $R^3$ hat, ausser Wasserstoff, oder wobei $R^2$ und $R^3$ zusammen mit dem N-Atom einen Pyrrolidin-, Pyrazolin-, Piperidin-, Piperazin- oder Morpholinring darstellen, welcher durch $C_{1-4}$-Alkyl oder Phenyl substituiert sein kann;

dadurch **gekennzeichnet,** dass ein Chlor-nitro-benzolsulfonylchlorid der Formel

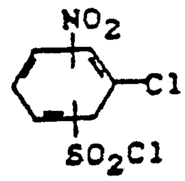

mit einem Amin oder Alkoholat der Formel H-$R^1$, in der $R^1$ die zuvor angegebene Bedeutung hat, in das entsprechende Chlor-nitro-sulfonsäurederivat umgewandelt wird, und eine Chlorid/Sulfid-Austauschreaktion anschliessend in Anwesenheit von $Na_2S$ durchgeführt wird, gefolgt von Oxidation, wobei ein symmetrisches Disulfid erhalten wird, oder dass ein durch Chlorid/Sulfid-Austauschreaktion erhaltenes Mercapto-nitro-benzol-sulfonsäurederivat der Formel

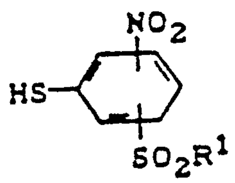

in der $R^1$ die zuvor angegebene Bedeutung hat, mit einem substituierten Phenylsulfenylchlorid der Formel

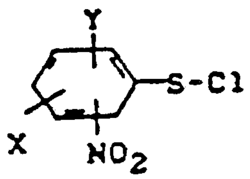

in der X und Y die zuvor angegebenen Bedeutungen haben, umgesetzt wird, wodurch ein asymmetrisches Disulfid erhalten wird.